(19) Europäisches Patentamt
European Patent Office
Office européen des brevets

(11) Veröffentlichungsnummer: **0 248 245 B1**

(12) # EUROPÄISCHE PATENTSCHRIFT

(45) Veröffentlichungstag der Patentschrift: **24.07.91**

(51) Int. Cl.[5]: **C07C 309/30**, C07C 311/15

(21) Anmeldenummer: **87107040.5**

(60) Veröffentlichungsnummer der früheren Anmeldung nach Art. 76 EPÜ: **0 102 925**

(22) Anmeldetag: **26.08.83**

(54) **2-Fluoralkenyl-phenylsulfonsäure-Derivate.**

(30) Priorität: **01.09.82 CH 5201/82**

(43) Veröffentlichungstag der Anmeldung:
**09.12.87 Patentblatt 87/50**

(45) Bekanntmachung des Hinweises auf die Patenterteilung:
**24.07.91 Patentblatt 91/30**

(84) Benannte Vertragsstaaten:
**AT BE CH DE FR GB IT LI NL SE**

(56) Entgegenhaltungen:
**EP-A- 0 044 210**
**EP-A- 0 044 807**

(73) Patentinhaber: **CIBA-GEIGY AG**
**Klybeckstrasse 141**
**CH-4002 Basel(CH)**

(72) Erfinder: **Meyer, Willy**
**Talweg 49**
**CH-4125 Riehen(CH)**
Erfinder: **Reinehr, Dieter, Dr.**
**Wolfsheule 10**
**W-7842 Kandern(DE)**
Erfinder: **Oertle, Konrad, Dr.**
**Vogesenstrasse 10**
**CH-4106 Therwil(CH)**
Erfinder: **Schurter, Rolf, Dr.**
**Holzmattstrasse 45**
**CH-4102 Binningen(CH)**

EP 0 248 245 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 2-Fluoralkenyl-phenylsulfonsäure-Derivate als Zwischenprodukte für herbizid wirksame und pflanzenwuchsregulierende N-Phenylsulfonyl-N'-pyrimidinyl- und -triazinylharnstoffe, sowie Verfahren zu ihrer Herstellung.

Herbizid und pflanzenwuchsregulierend wirksame N-Phenylsulfonyl-N'-pyrimidinyl- und -triazinyl-harnstoffe der allgemeinen Formel I

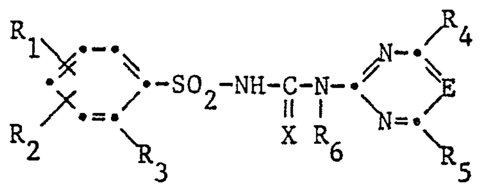 (I),

worin

| | |
|---|---|
| $R_1$ | Wasserstoff, Halogen, Nitro, Amino, $C_1$-$C_5$-Alkyl, $C_1$-$C_4$-Halogenalkyl oder einen Rest -Q-$R_7$, -CO-$OR_8$ oder -$(CO)_n$-$NR_9R_{10}$, |
| $R_2$ | Wasserstoff, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Halogen oder Alkoxyalkyl mit höchstens 4 Kohlenstoffatomen, |
| $R_3$ | einfach mehrfach durch Fluor substituiertes $C_2$-$C_{10}$-Alkenyl, dessen Doppelbindung direkt an den Phenylkern gebunden ist, |
| $R_4$ | $C_1$-$C_3$-Alkyl, $C_1$-$C_3$-Halogenalkyl, $C_1$-$C_3$-Alkoxy oder $C_1$-$C_3$-Halogenalkoxy, |
| $R_5$ | Wasserstoff, Halogen, -$NR_{13}R_{14}$, $C_1$-$C_3$-Alkyl, $C_1$-$C_3$-Halogenalkyl, $C_1$-$C_3$-Alkoxy oder $C_1$-$C_2$-Halogenalkoxy, |
| $R_6$ | Wasserstoff, $C_1$-$C_3$-Alkyl oder $C_1$-$C_3$-Alkoxy, |
| X | Sauerstoff oder Schwefel und |
| E | Stickstoff oder die Methingruppe bedeuten, wobei |
| $R_7$ | für gegebenenfalls durch Halogen oder $C_1$-$C_3$-Alkoxy substituiertes $C_1$-$C_4$-Alkyl oder $C_3$-$C_5$-Alkenyl, |
| $R_8$ | für $C_1$-$C_4$-Alkyl, $C_1$-$C_4$-Halogenalkyl oder $C_2$-$C_6$-Alkoxyalkyl, |
| $R_9$, $R_{10}$, $R_{13}$ und $R_{14}$ | unabhängig voneinander für Wasserstoff oder $C_1$-$C_3$-Alkyl, |
| Q | für Sauerstoff, Schwefel, die Sulfinyl- oder Sulfonylbrücke und |
| n | für Null oder eins stehen |

sowie Salze dieser Verbindungen bilden den Gegenstand unserer Europäischen Patentanmeldung EP-A-102925.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in der EP-Patentanmeldung 44210, in der DE-OS 2 715 786 oder im niederländischen Patent 121788 beschrieben.

In den Definitionen ist unter Alkyl geradkettiges oder verzweigtes Alkyl zu verstehen; z.B.: Methyl, Aethyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen: Methoxy, Aethoxy, n-Propyloxy, i-Propyloxy und die vier isomeren Butyloxyreste, insbesondere aber Methoxy, Aethoxy oder i-Propoxy.

Beispiele für Alkylthio sind Methylthio, Aethylthio, n-Propylthio, i-Propylthio und n-Butylthio, insbesondere aber Methylthio und Aethylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiel für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl, sowie isomere Pentinyl-oder Hexinylreste, vorzugsweise ist der Alkinylrest jedoch durch Propargyl oder 2- oder 3-Butinyl verkörpert.

Die EP-A-102925 umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Substituierte Alkenylreste, die der Definition des Substituenten $R_3$ der Formel I entsprechen, sind beispielsweise: 3-Fluor-1-propen-1-yl, 1-Fluormethyl-vinyl, 1-Difluormethyl-vinyl, 1-Trifluormethyl-vinyl, 3,3-Difluor-1-propen-1-yl, 3,3,3-Trifluor-1-propen-1-yl, 2,2-Difluorvinyl, Perfluorvinyl oder 3-Fluor-1-buten-1-yl.

Bevorzugt sind Fluoralkenylreste mit höchstens 5 Kohlenstoffatomen. Solche bevorzugten Alkenylreste sind 3,3,3-Trifluor-1-propen-1-yl und 3,3-Difluor-1-propen-1-yl.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den Verbindungen der Formel I sind die Verbindungen bevorzugt, in denen entweder

a) X Sauerstoff bedeutet oder
b) $R_4$ und $R_5$ zusammen nicht mehr als 4 Kohlenstoffatome enthalten oder
c) $R_6$ Wasserstoff bedeutet oder
d) $R_1$ und $R_2$ Wasserstoff bedeuten oder
e) $R_3$ 2 bis 4 Kohlenstoffatome enthält.

Eine ganz besonders bevorzugte Untergruppe von Verbindungen der Formel I bilden diejenigen Verbindungen, in denen X für Sauerstoff und $R_1$, $R_2$ und $R_6$ für Wasserstoff stehen und $R_4$ und $R_5$ zusammen nicht mehr als 4 Kohlenstoffatome enthalten und $R_3$ 2 bis 4 Kohlenstoffatome enthält.

Als bevorzugte Einzelverbindungen sind zu nennen:
N-[2-(3,3,3-Trifluor-1-propen-1-yl)-phenyl-sulfonyl]-N'-(4-methoxy-6-methyl-1,3,5-triazin-2-yl)-harnstoff und
N-[2-(3,3,3-Trifluor-1-propen-1-yl)-phenyl-sulfonyl]-N'-(4,6-dimethoxy-1,3,5-triazin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

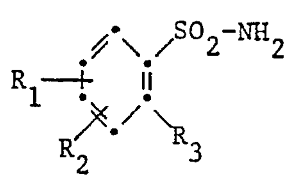

(II),

worin $R_1$, $R_2$ und $R_3$ die unter Formel I gegebene Bedeutung haben, in Gegenwart einer Base mit einem N-Pyrimidinyl- oder -Triazinylcarbamat der Formel III

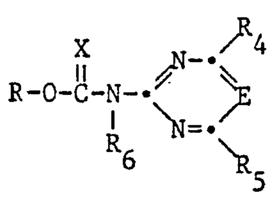

(III),

worin E, $R_4$, $R_5$, $R_6$ und X die unter Formel I gegebene Bedeutung haben und R für Phenyl, Alkyl oder substituiertes Phenyl steht, umsetzt.

Nach einem weiteren Verfahren gelangt man zu Verbindungen der Formel I, indem man ein Phenylsulfonylisocyanat oder -isothiocyanat der Formel IV

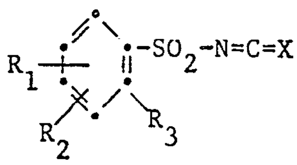

(IV),

worin $R_1$, $R_2$, $R_3$ und X die unter Formel I gegebene Bedeutung haben, gegebenenfalls in Gegenwart einer Base, mit einem Amin der Formel V

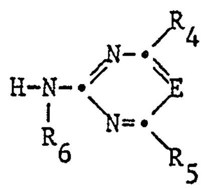

(V),

worin E, $R_4$, $R_5$ und $R_6$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

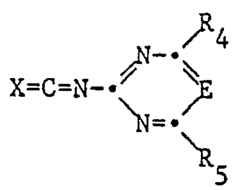

(VI),

worin E, $R_4$, $R_5$ und X die unter Formel I gegebene Bedeutung haben, umsetzt.

Schliesslich kann man die Verbindungen der Formel I auch erhalten, indem man ein N-Phenylsulfonylcarbamat der Formel VII

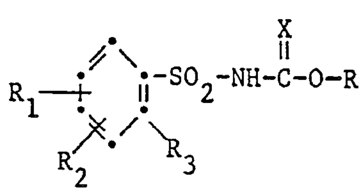

(VII),

worin $R_1$, $R_2$, $R_3$ und X die unter Formel I angegebene Bedeutung haben und R für Phenyl, Alkyl oder substituiertes Phenyl steht, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen -20° und +120° C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch

Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen, gereinigt werden.

Die Wirkstoffe der Formel I sind stabile Verbindungen. Ihre Handhabung bedarf keiner vorsorglichen Massnahmen.

Die Ausgangsstoffe der Formeln II, IV und VII sind zum Teil neu und können nach den folgenden Methoden hergestellt werden und wurden speziell für die Synthese der Verbindungen der Formel I entwickelt. Diese neuen Verbindungen werden in der allgemeinen Formel IIa zusammengefasst. Sie bilden zusammen mit ihren Herstellungsverfahren den Gegenstand der vorliegenden Teilanmeldung.

Die erfindungsgemässen neuen Zwischenprodukte entsprechen der Formel IIa

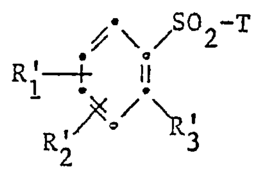

(IIa),

worin

| | |
|---|---|
| T | Hydroxyl, -OM, $-O\frac{M_1}{2}$, Cl, -N=C=O oder $-NHT_4$, M ein Alkalimetallatom, $M_1$ ein Erdalkalimetallatom, |
| $R_1'$ | Wasserstoff, Halogen, $C_1$-$C_5$-Alkyl, $C_1$-$C_4$-Halogenalkyl, $C_1$-$C_4$-Alkoxy, $-COOR_8$, $-CONR_9R_{10}$ oder $-NO_2$, |
| $R_2'$ | Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder Alkoxyalkyl mit höchstens 4 Kohlenstoffatom und |
| $R_3'$ | eine Gruppe $-C(T_1)=C(T_2)(T_3)$ bedeuten, |
| $T_1$ | Wasserstoff, |
| $T_2$ | ein- oder mehrfach durch Fluor substituiertes $C_1$-$C_8$-Alkyl, |
| $T_3$ | Wasserstoff oder gegebenenfalls durch Fluoratome substituiertes $C_1$-$C_5$-Alkyl, |
| $T_4$ | Wasserstoff, -CO-NH-$C_1$-$C_4$-Alkyl, -COO-$C_1$-$C_4$-Alkyl oder -COO-Phenyl, |
| $R_8$ | $C_1$-$C_4$-Alkyl, $C_1$-$C_4$-Halogenalkyl oder $C_2$-$C_6$-Alkoxyalkyl, und |
| $R_9$ und $R_{10}$ | unabhängig voneinander Wasserstoff oder $C_1$-$C_3$-Alkyl bedeuten, wobei durch $T_2$ und $T_3$ dargestellte Alkylgruppen zusammen höchstens 8 Kohlenstoffatome aufweisen. |

Die erfindungsgemässen Zwischenprodukte der Formel IIa können dadurch hergestellt werden, dass man ein Amin der Formel VIII

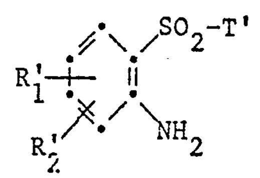

(VIII)

zu einem Diazoniumsalz der Formel IX

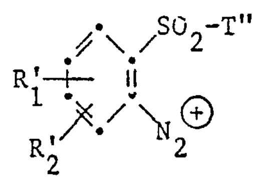

(IX)

diazotiert, dieses in Gegenwart von Palladium-Katalysatoren, die unter den Reaktionsbedingungen Pd(O)-Verbindungen bilden, und gegebenenfalls in Gegenwart einer Base mit einer Verbindung der Formel X

$H - R_3'$ (X)

zu einer Verbindung der Formel XI

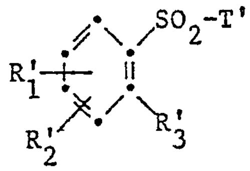

(XI)

umsetzt, die Verbindung der Formel XI in an sich bekannter Weise durch Behandlung mit einem Chlorierungsmittel, wie Thionylchlorid oder $PCl_5$ in das entsprechende Sulfonylchlorid überführt, dieses mit Ammoniak behandelt und so erhaltene Verbindungen der Formel IIa, worin T für $-NH_2$ steht, gegebenenfalls in Verbindungen der Formel IIa, worin $T_4$ nicht Wasserstoff bedeutet, überführt, indem man die erhaltenen Sulfonamide mit den Acylierungsmitteln $O=C=N=C_1$-$C_4$-Alkyl, ClCONH-$C_1$-$C_4$-Alkyl, ClCOO-$C_1$-$C_4$-Alkyl, ClCOO-Phenyl oder mit von $C_1$-$C_4$-Alkylisocyanaten, wobei T′ OH, OM oder

$$O\frac{M_1}{2}$$

und T″ OM oder

$$O\frac{M_1}{2}$$

bedeuten und M, $M_1$, $R_1'$, $R_2$, $R_3'$ die unter Formel IIa angegebene Bedeutung haben, umsetzt.

Die Diazotierung kann nach an sich bekannten Methoden in saurem wässrigem Medium, wie wässriger HCl, $H_2SO_4$, $H_2O$/Essigsäure, durchgeführt werden. Die Umsetzung der Diazoniumsalze mit den Olefinen wird zweckmässig in Gegenwart eines inerten organischen Lösungsmittels vorgenommen. Dazu eignen sich z.B. gegebenenfalls chlorierte aliphatische Monocarbonsäuren, besonders Essigsäure, Chloressigsäure, Dichloressigsäure oder Trifluoressigsäure, Aceton, Dichlormethan und Acetonitril oder Gemische der genannten Lösungsmittel. Bevorzugt verwendet man Essigsäure.

Als Palladium-Katalysatoren und Basen können z.B. solche der in der EP-Patentanmeldung 40177 beschriebenen Art verwendet werden. Bevorzugte Palladium-Katalysatoren sind $PdCl_2$, $[PdCl_4]Na_2$ oder $[PdCl_4]Li_2$, vor allem jedoch Bis-(dibenzylidenaceton)Palladium(O). Als Basen verwendet man vorzugsweise Alkalimetallcarboxylate, wie Natriumacetat. Eine Isolierung der Diazoniumsalze der Formel IX und der Sulfonylchloride ist im allgemeinen nicht erforderlich. Wird die Diazotierung in Gegenwart von Essigsäure und von nur einem Aequivalent einer starken Säure durchgeführt und werden die erhaltenen Diazoniumsalze ohne Isolierung weiterverwendet, so kann im allgemeinen bei der Umsetzung mit den Olefinen der Formel X auf den Zusatz von Base verzichtet werden.

Verbindungen der Formel IIa, worin $R_1'$ und $R_2'$ ungleich Brom bzw. Jod sind, können auch dadurch hergestellt werden, dass man eine Verbindung der Formel XII

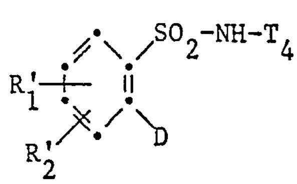

(XII)

worin $R_1'$, $R_2'$, und $T_4$ die unter Formel IIa angegebene Bedeutung haben und D Brom oder Jod bedeutet,

in Gegenwart einer gegebenenfalls arsen- oder phosphorhaltigen Palladium-Verbindung als Katalysator und einer Base mit einem Olefin der obigen Formel X umsetzt.

Als Palladium-Katalysatoren und Basen für diese Herstellungsvariante eignen sich beispielsweise Verbindungen der in der US-PS 3.922.299 beschriebenen Art. Als Katalysatoren verwendet man bevorzugt Gemische aus Palladiumacetat und Triphenylphosphin oder Tri-o-tolylphosphin. Stellt D in Formel XII Jod dar, so können auch arsen- bzw. phosphorfreie Palladium-Verbindungen, vor allem Palladiumacetat, eingesetzt werden. Als Basen eignen sich vor allem Trialkylamine, besonders Triäthylamin oder Tri-n-butylamin, und Alkalimetallcarboxylate, vor allem Natriumacetat.

Die Palladiumkatalysatoren werden bei beiden Verfahrensvarianten zweckmässig in einer Menge von etwa 0,01 bis 5 Mol%, bezogen auf das Diazoniumsalz der Formel IX bzw. das Halogenbenzol der Formel XII, verwendet.

Die Umsetzung der Halogenbenzole der Formel XII mit den Olefinen der Formel X wird ebenfalls zweckmässig in Gegenwart eines inerten organischen Lösungsmittels vorgenommen, z.B. gegebenenfalls chlorierten aromatischen Kohlenwasserstoffen, wie Toluol, Xylolen oder Chlorbenzol, oder N,N-Dialkylamiden von aliphatischen Monocarbonsäuren der oben erwähnten Art, besonders N,N-Dimethylformamid.

Die Ausgangsprodukte der Formeln III, V, VI, VIII, X und XII sind bekannt oder können auf an sich bekannte Weise hergestellt werden.

Die Verbindungen der Unterformeln IV und VII können in an sich bekannter Weise aus den Verbindungen der Formel II, welche ihrerseits ebenfalls einen Teil der Formel IIa bildet, erhalten werden.

So können die Phenylsulfonylisocyanate der Formel IV durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in "Neuere Methoden der präparativen organischen Chemie", Band VI, 211-229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 299, 174 (1966) beschrieben. Die N-Phenylsulfonylcarbamate der Formel VII werden durch Umsetzung der Sulfonamide der Formel II mit Diphenylcarbonat in Gegenwart einer Base erhalten. Aehnliche Verfahren sind in der japanischen Patentschrift 61 169 erwähnt.

Zwischenprodukte der Formeln III und VI können nach bekannten Methoden aus entsprechenden Verbindungen der Formel V erhalten werden.

Neue Fluoralkoxy-aminopyrimidine und -triazine der Formel V und ihre Herstellung sowie die Herstellung entsprechender Verbindungen der Formel III und VI daraus werden in der EP-A-70804 beschrieben.

Durch Umsetzung von Aminen der Formel V mit Oxalylchlorid in chlorierten Kohlenwasserstoffen als Lösungsmittel, lassen sich Isocyanate der Formel VI herstellen. Amine der Formel V sind bekannt und zum Teil im Handel erhältlich oder sie können nach bekannten Methoden hergestellt werden, siehe "The Chemistry of Heterocyclic Compounds" Band XIV, Interscience Publishers, New York, London.

In Formel IIa ist T vorzugsweise $-NH_2$.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Die bevorzugte Kultur ist Getreide wie Weizen, Gerste und Roggen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragsteigerung mit Wachstumshemmern beruht darauf, dass die Nährstoffe in stärkerem Masse der Blüten- und Fruchtbildung zugute kommen, während das vegetative Wachstum eingeschränkt wird.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt

werden, so dass zwar die Bodenerosion zwischen den Kulturpflanzen verhindert wird, die "cover crops" jedoch nicht zur Konkurrenz für die Kultur werden können.
Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Regulierung des Pflanzenwuchses von monokotylen und dikotylen Pflanzen, insbesondere Gräsern, Nutzpflanzen, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulver, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder-äthyläther, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorganulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und eine Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Rigdewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

<u>Emulgierbare Konzentrate</u>

| | |
|---|---|
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

<u>Stäube</u>

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

Suspension-Konzentrate

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:

Beispiel 1: 2-(3,3,3-Trifluor-1-propen-1-yl)-phenylsulfonamid.

a) Orthanilsäure-Diazoniumsalz.

34,64 g (0,2 Mol) Orthanilsäure werden in 30 ml Wasser aufgeschlämmt, mit 62,3 ml Borfluorwasserstoffsäure (50%ig; 0,5 Mol) versetzt und auf 0-5°C gekühlt. Innerhalb einer Stunde werden bei dieser Temperatur tropfenweise 13,8 g (0,2 Mol) Natriumnitrit, gelöst in 20 ml Wasser, unter Rühren zugetropft. Nach zusätzlichem Rühren während 30 Minuten werden 100 ml Diäthyläther zugegeben und die abgekühlte Suspension wird filtriert. Das isolierte feste Diazoniumsalz wird zusätzlich mit 100 ml eines 1:1 Gemisches aus Essigsäure und Diäthyläther und anschliessend mit nochmals 100 ml Diäthyläther gewaschen. Nach kurzem Trocknen an der Luft erhält man 34 g Diazoniumsalz; Ausbeute 92% d.Th. Ohne Ausbeuteeinbusse kann anstelle von Borfluorwasserstoffsäure konz. Salzsäure oder konz. Schwefelsäure verwendet werden.

b) 8,06 g (0,0438 Mol) des Diazoniumsalzes der Orthanilsäure werden in 150 ml Essigsäure aufgeschlämmt und mit 3,59 g (0,0438 Mol) Natriumacetat versetzt. Nach Zugabe von 0,256 g ($4,38 \times 10^{-4}$ Mol) Bis-(benzylidenaceton)Palladium(O) wird das Reaktionsgefäss (250 ml-Fischer-Porter-Flasche/Druckapparatur) einmal evakuiert. Dann werden 6 g (0,0625 Mol) 3,3,3-Trifluorpropen aufgepresst. Unter starkem Rühren setzt die Reaktion sofort unter Stickstoffentwicklung (Druckanstieg bis ca. 8 bar) und Temperaturanstieg (max. ca. 45°C) ein und ist nach ca. 90 Minuten beendet. Das Lösungsmittel wird im Rotationsverdampfer abgezogen.

Der erhaltene Rückstand (9,96 g) wird in 50 ml N,N-Dimethylformamid gelöst und tropfenweise mit 6,61 ml (0,0909 Mol) Thionylchlorid versetzt. Nach 2 Stunden Rühren bei Raumtemperatur wird das Reaktionsgemisch auf Eis gegossen und das entstandene Sulfonylchlorid wird abfiltriert. Man erhält 6,37 g Sulfonylchlorid, die direkt in 20 ml Essigsäureäthylester gelöst und bei 0-5°C in 25 ml konz. Ammoniak eingetropft werden. Nach vollständiger Umsetzung des Sulfonylchlorids wird mit Wasser verdünnt und mehrmals mit Essigsäureäthylester extrahiert. Aus den getrockneten Essigsäureäthylester-Phasen werden 5,35 g Produkt isoliert, das durch Chromatographie an Kieselgel oder durch Umkristallisieren aus Essigsäureäthylester/n-Hexan gereinigt wird. Man erhält 5,08 g (0,0239 Mol) 2-(3,3,3-Trifluor-1-propen-1-yl)-phenylsulfonamid; Ausbeute 54% d.Th., bezogen auf das Diazoniumsalz, Smp. 153-154 °C.

Analyse: $C_9H_8F_3NO_2S$ (251,22)

Ber. C 43,03 H 3,21 F 22,69 N 5,58 S 12,76

Gef. C 42,99 H 3,29 F 22,73 N 5,53 S 12,97.

Beispiel 2: 2-(2-Perfluorhexyl-vinyl)-phenylsulfonamid.

16,4 g (0,089 Mol) des Diazoniumsalzes der Orthanilsäure werden in 100 ml Essigsäure aufgeschlämmt. Dazu gibt man 7,31 g (0,089 Mol) Natriumacetat, gefolgt von 0,5116 g (8,9 x $10^{-4}$ Mol) Bis-(benzylidenaceton)Palladium(O). Bei Raumtemperatur lässt man 34,61 g (0,098 Mol)Perfluorhexyläthylen (85%ig) zutropfen. Unter $N_2$-Entwicklung setzt sofort die exotherme Reaktion ein. Die Reaktionstemperatur wird durch externe Kühlung und Aenderung der Tropfgeschwindigkeit auf 30-40 °C gehalten. Nach Beendigung der Reaktion wird die Essigsäure anschliessend am Rotationsverdampfer unter Zusatz von Toluol möglichst vollständig entfernt. Der erhaltene Rückstand wird wie in Beispiel 4 beschrieben ohne Reinigung über das Sulfonylchlorid in das entsprechende Sulfonamid übergeführt. Nach Chromatographie an Kieselgel erhält man 30,28 g (0,059 Mol) 2-(2-Perfluorhexylvinyl)-phenylsulfonamid in einer Ausbeute von 67% d.Th. (bezogen auf das Diazoniumsalz); Smp. 60-61 °C.

Beispiel 3: N-[2-(3,3,3-Trifluor-1-propen-1-yl)-phenyl-sulfonyl]-N'-(4-methoxy-6-methyl-1,3,5-triazin-2-yl)-harnstoff.

Eine Lösung von 5,0 g 2-(3,3,3-Trifluor-1-propen-1-yl)-phenylsulfonamid und 3,3 g 1,5-Diazabicyclo-[5,4,0]undec-5-en in 80 ml Dioxan wird mit 5,2 g N-(4-Methoxy-6-methyl-1,3,5-triazin-2-yl)-phenylcarbonat versetzt und für 3 Stunden bei 20 ° bis 25 °C gerührt. Anschliessend wird die klare Reaktionslösung in 300 ml Wasser aufgenommen und mit 2 N Salzsäure bis auf pH 4-5 angesäuert. Der ausgefallene harzartige Niederschlag wird mit Aethylacetat aus der wässrigen Phase extrahiert. Der organische Extrakt wird getrocknet und eingedampft. Der ölige Rückstand wird aus Aceton/Aether-Gemisch (1:10) kristallisiert. Man erhält so 7,2 g N-[2-(3,3,3-Trifluor-1-propen-1-yl)-phenyl-sulfonyl]-N'-(4-methoxy-6-methyl-1,3,5-triazin-2-yl)-harnstoff, Smp. 159-160 °C.

Beispiel 4: N-[2-(3,3,3-Trifluor-1-propen-1-yl)-phenyl-sulfonyl)-N'-(4-methoxy-6-methyl-1,3,5-triazin-2-yl)-harnstoff.

a) 5,0 g 2-(3,3,3,-Trifluor-1-propen-1-yl)-phenyl-sulfonamid und 1,7 g Methylisocyanat werden in 25 ml Methylenchlorid suspendiert und tropfenweise innerhalb von 10 Minuten mit 3,0 g Triäthylamin versetzt. Es entsteht eine klare Lösung. Nach dem Eindampfen der Lösung wird der Rückstand in 5%iger Natriumcarbonatlösung gelöst und von den unlöslichen Bestandteilen abfiltriert. Durch Ansäuern der klaren Lösung mit 10%iger Salzsäure erhält man als farblosen Niederschlag 5,9 g N-[2-(3,3,3-Trifluor-1-propen-1-yl)-phenyl-sulfonyl]-N'-methyl-harnstoff, Smp. 190-192 °C.

b) 5,9 g N-[2-(3,3,3-Trifluor-1-propen-1-yl)-phenyl-sulfonyl]-N'-methyl-harnstoff werden in 100 ml Chlorbenzol suspendiert. Durch Rückflusskochen am Wasserabscheider wird die Lösung getrocknet. Anschliessend leitet man bei einer Temperatur von 120-130 °C innerhalb von 20 Minuten 6,0 g Phosgen in das Reaktionsgemisch ein. Durch Abdampfen des Lösungsmittels erhält man als gelbliches Oel 5,6 g 2-(3,3,3-Trifluor-1-propen-1-yl)-phenyl-sulfonylisocyanat.

c) 5,6 g 2-(3,3,3-Trifluor-1-propen-1-yl)-phenyl-sulfonylisocyanat und 2,5 g 2-Amino-4-methoxy-6-methyl-1,3,5-triazin werden in 60 ml absolutem Dioxan für 3 Stunden bei 70-80 °C gerührt. Nach dem Abkühlen auf 20 °C wird das Reaktionsgemisch filtriert und die erhaltene klare Lösung auf ein Viertel des Volumens eingedampft. Nach der Zugabe von 50 ml Aether kristallisieren aus der Lösung 5,0 g N-[2-(3,3,3-Trifluor-1-propen-1-yl)-phenyl-sulfonyl]-N'-(4-methoxy-6-methyl-1,3,5-triazin-2-yl)-harnstoff aus, Smp. 154-155 °C.

In analoger Weise erhält man die in der anschliessenden Tabelle aufgelisteten Zwischen- und Endprodukte.

Tabelle 1:

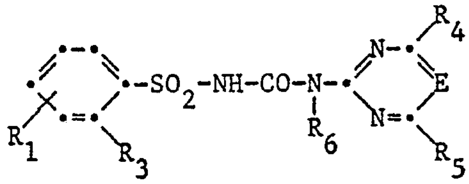

| Nr. | $R_1$ | $R_3$ | $R_4$ | $R_5$ | $R_6$ | E | phys.Daten |
|---|---|---|---|---|---|---|---|
| 1.1 | H | $-CH=CH-CF_3$ | $CH_3$ | $OCH_3$ | H | N | Smp.159-160°C |
| 1.2 | H | $-CH=CH-CF_3$ | $OCH_3$ | $OCH_3$ | H | N | Smp.185-186°C |
| 1.3 | H | $-CH=CH-CF_3$ | $CH_3$ | $OCH_3$ | H | CH | Smp.166-169°C |
| 1.4 | H | $-CH=CH-CF_3$ | $CH_3$ | $CH_3$ | H | CH | Smp.186-189°C |
| 1.5 | H | $-CH=CH-CF_3$ | $CH_3$ | $OCHF_2$ | H | CH | Smp.178-179°C |
| 1.6 | H | $-CH=CH-CF_3$ | $OCH_3$ | $OCH_3$ | H | CH | Smp.185-186°C |
| 1.7 | H | $-CH=CH-CF_3$ | $OCH_3$ | $-OCH_2-CF_3$ | H | N | Smp.151-152°C |
| 1.8 | H | $-CH=CH-CF_3$ | $OCH_3$ | $-N(CH_3)_2$ | H | N | Smp.174-175°C |
| 1.9 | H | $-CH=CH-CF_3$ | $OCH_3$ | $-N(CH_3)_2$ | H | CH | |
| 1.10 | H | $-CH=CH-CF_3$ | Cl | $OCHF_2$ | H | CH | |
| 1.11 | H | $-CH=CH-CF_3$ | $OCHF_2$ | $-N(CH_3)_2$ | H | CH | |
| 1.12 | H | $-CH=CH-CF_3$ | Cl | $OCH_3$ | H | CH | Smp.174-175°C |

Tabelle 1 (Fortsetzung)

| Nr. | $R_1$ | $R_3$ | $R_4$ | $R_5$ | $R_6$ | E | phys.Daten |
|---|---|---|---|---|---|---|---|
| 1.13 | H | $-CH=CH-CF_3$ | $OCH_3$ | $OCH_3$ | $CH_3$ | CH | |
| 1.14 | H | $-CH=CH-CH_2F$ | $CH_3$ | $CH_3$ | H | CH | |
| 1.15 | H | $-CH=CH-CH_2F$ | $CH_3$ | $OCH_3$ | H | CH | |
| 1.16 | H | $-CH=CH-CH_2F$ | $OCH_3$ | $OCH_3$ | H | CH | |
| 1.17 | H | $-CH=CH-CH_2F$ | $CH_3$ | $OCH_3$ | H | N | |
| 1.18 | H | $-CH=CH-CH_2F$ | $OCH_3$ | $OCH_3$ | H | N | |
| 1.19 | H | $-CH=CH-CHF_2$ | $CH_3$ | $CH_3$ | H | CH | |
| 1.20 | H | $-CH=CH-CHF_2$ | $CH_3$ | $OCH_3$ | H | CH | |
| 1.21 | H | $-CH=CH-CHF_2$ | $OCH_3$ | $OCH_3$ | H | CH | |
| 1.22 | H | $-CH=CH-CHF_2$ | $CH_3$ | $OCH_3$ | H | N | |
| 1.23 | H | $-CH=CH-CHF_2$ | $OCH_3$ | $OCH_3$ | H | N | |
| 1.24 | 6-F | $-CH=CH-CF_3$ | $CH_3$ | $CH_3$ | H | CH | |
| 1.25 | 6-F | $-CH=CH-CF_3$ | $CH_3$ | $OCH_3$ | H | CH | |
| 1.26 | 6-F | $-CH=CH-CF_3$ | $OCH_3$ | $OCH_3$ | H | CH | |
| 1.27 | 6-F | $-CH=CH-CF_3$ | $CH_3$ | $OCH_3$ | H | N | |
| 1.28 | 6-F | $-CH=CH-CF_3$ | $OCH_3$ | $OCH_3$ | H | N | |
| 1.29 | 5-F | $-CH=CH-CF_3$ | $CH_3$ | $CH_3$ | H | CH | |
| 1.30 | 5-F | $-CH=CH-CF_3$ | $OCH_3$ | $OCH_3$ | H | CH | |
| 1.31 | 5-F | $-CH=CH-CF_3$ | $CH_3$ | $OCH_3$ | H | N | |
| 1.32 | 5-F | $-CH=CH-CF_3$ | $OCH_3$ | $OCH_3$ | H | N | |
| 1.33 | 3-F | $-CH=CH-CF_3$ | $CH_3$ | $CH_3$ | H | CH | |
| 1.34 | 3-F | $-CH=CH-CF_3$ | $CH_3$ | $OCH_3$ | H | CH | |
| 1.35 | 3-F | $-CH=CH-CF_3$ | $OCH_3$ | $OCH_3$ | H | CH | |
| 1.36 | 3-F | $-CH=CH-CF_3$ | $CH_3$ | $OCH_3$ | H | N | |
| 1.37 | 3-F | $-CH=CH-CF_3$ | $OCH_3$ | $OCH_3$ | H | N | |

Tabelle 1 (Fortsetzung)

| Nr. | $R_1$ | $R_3$ | $R_4$ | $R_5$ | $R_6$ | E | phys.Daten |
|---|---|---|---|---|---|---|---|
| 1.38 | H | $-CH=CH-C_6F_{13}-n$ | $CH_3$ | $CH_3$ | H | CH | Smp.134-138°C |
| 1.39 | H | $-CH=CH-C_6F_{13}-n$ | $CH_3$ | $OCH_3$ | H | CH | Smp.114-116°C |
| 1.40 | H | $-CH=CH-C_6F_{13}-n$ | $OCH_3$ | $OCH_3$ | H | CH | |
| 1.41 | H | $-CH=CH-C_6F_{13}-n$ | $CH_3$ | $OCH_3$ | H | N | Smp.133-136°C |
| 1.42 | H | $-CH=CH-C_6F_{13}-n$ | $OCH_3$ | $OCH_3$ | H | N | |
| 1.43 | H | $-CH=CH-C_3F_7-n$ | $CH_3$ | $OCH_3$ | H | N | Smp. 128-130°C |
| 1.44 | H | $-CH=CH-CF_2-CH_3$ | $CH_3$ | $OCH_3$ | H | N | Smp. 162-163°C |
| 1.45 | H | $-CH=C(CF_3)-CH_2CF_3$ | $CH_3$ | $OCH_3$ | H | N | Smp. 164-165°C |
| 1.46 | H | $-CH=C(CH_3)-CF_3$ | $CH_3$ | $OCH_3$ | H | CH | Smp. 161-162°C |
| 1.47 | H | $-CH=CH-CF_3$ | $CH_3$ | $OC_2H_5$ | H | N | Smp. 146-149°C |
| 1.48 | H | $-CH=CHC_3F_7n$ | $OCH_3$ | $OCH_3$ | H | N | |
| 1.49 | H | $-CH=CHC_3F_7n$ | $CH_3$ | $OCH_3$ | H | CH | |
| 1.50 | H | $-CH=CHC_3F_7n$ | $OCH_3$ | $OCH_3$ | H | CH | |
| 1.51 | H | $-CH=CHCF_2CH_3$ | $OCH_3$ | $OCH_3$ | H | N | |
| 1.52 | H | $-CH=CH-CF_2-CH_3$ | $OCH_3$ | $CH_3$ | H | CH | |
| 1.53 | H | $-CH=CH-CF_2CH_3$ | $OCH_3$ | $OCH_3$ | H | CH | |
| 1.54 | H | $-CH=CH-CF_3$ | $OCH_3$ | $OCHF_2$ | H | CH | |
| 1.55 | H | $-CH=CH-CF_3$ | $OCHF_2$ | $OCHF_2$ | H | CH | |
| 1.56 | H | $-CH=CH-CF_3$ | $OCH_3$ | $OCH_2CH_3$ | H | N | |
| 1.57 | H | $-CH=CH-CF_3$ | $OCH_2CH_3$ | $OCH_2CH_3$ | H | N | |
| 1.58 | H | $-CH=CH-CF_3$ | $CH_2CH_3$ | $OCH_3$ | H | N | |
| 1.59 | $5-CH_3$ | $-CH=CH-CF_3$ | $CH_3$ | $OCH_3$ | H | N | Smp. 161-163°C |
| 1.60 | $5-CH_3$ | $-CH=CH-CF_3$ | $OCH_3$ | $OCH_3$ | H | N | |
| 1.61 | $5-CH_3$ | $-CH=CH-CF_3$ | $CH_3$ | $OCH_3$ | H | CH | |
| 1.62 | $5-CH_3$ | $-CH=CH-CF_3$ | $OCH_3$ | $OCH_3$ | H | CH | |
| 1.63 | $5-NO_2$ | $-CH=CH-CF_3$ | $CH_3$ | $OCH_3$ | H | N | Smp. 170-172°C |
| 1.64 | $5-NO_2$ | $-CH=CH-CF_3$ | $OCH_3$ | $OCH_3$ | H | N | |
| 1.65 | $5-NO_2$ | $-CH=CH-CF_3$ | $CH_3$ | $OCH_3$ | H | CH | |
| 1.66 | $5-NO_2$ | $-CH=CH-CF_3$ | $OCH_3$ | $OCH_3$ | H | CH | |

Tabelle 2:

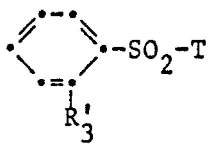

| Nr. | $R'_3$ | T | phys.Daten |
|---|---|---|---|
| 2.1 | $-CH=CH-CF_3$ | $NH_2$ | Smp.153-154°C |
| 2.2 | $-CH=CH-CF_3$ | $-NH-CO-NHCH_3$ | Smp.190-192°C |
| 2.3 | $-CH=CH-CF_3$ | $-N=C=O$ | Oel |
| 2.4. | $-CH=CH-CF_3$ | $-NH-CO-OC_6H_5$ | |
| 2.5 | $-CH=CH-C_6F_{13}-n$ | $NH_2$ | Smp. 60-61°C |
| 2.6 | $-CH=CH-C_3F_7-n$ | $NH_2$ | Smp. 63-64°C |
| 2.7 | $-CH=CH-CF_2CH_3$ | $NH_2$ | Smp. 105-106°C |
| 2.8 | $-CH=C(CF_3)-CH_2-CF_3$ | $NH_2$ | |
| 2.9 | $-CH=C(CH_3)-CF_3$ | $NH_2$ | |
| 2.10 | $-CH=CH-CF_3$ | Cl | Smp. 43-44°C |
| 2.11 | $-CH=CH-C_3F_7-n$ | Cl | |

Tabelle 3:

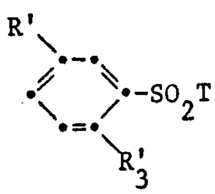

| Nr. | $R_1$ | $R'_3$ | T | phys. Daten |
|---|---|---|---|---|
| 3.1 | $CH_3$ | $-CH=CH-CF_3$ | $NH_2$ | Smp. 153-154°C |
| 3.2 | $NO_2$ | $-CH=CH-CF_3$ | $NH_2$ | Smp. 176-178°C |
| 3.3 | $C_2H_5$ | $-CH=CH-CF_3$ | $NH_2$ | |
| 3.4 | $C_2H_5$ | $-CH=CH-CF_3$ | $-N=C=O$ | |

Formulierungsbeispiele für Wirkstoffe der Formel I (% = Gewichtsprozent)

a) Spritzpulver

| | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsion-Konzentrat

| | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37%ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5 % |
| Isopropylamin | 1 % |
| Octylphenolpolyäthylenglykoläther (78 Mol AeO) | 3 % |
| Wasser | 91 % |

Biologische Beispiele

Herbizidwirkung vor dem Auflaufen der Pflanzen

Kunststofftöpfe werden mit expandiertem Vermiculit (Dichte: o,135 g/$cm^3$, Wasserabsorptionsvermögen: 0.565 l/l) gefüllt. Nach dem Sättigen des nicht adsorptiven Vermiculits mit einer wässrigen Wirkstoffemulsion in deionisiertem Wasser, die die Wirkstoffe der Formel I in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70% gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5% eines handelsüblichen Flüssigdüngers (® Greenzit) zugesetzt. 12 Tage nach der Aussaat wird der Versuch ausgewertet und die Wirkung auf die Versuchspflanzen nach dem folgenden Massstab bewertet:

1: Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze / Wirkstoff Nr. | Nasturtium | Stellaria | Agrostis | Digitaria |
|---|---|---|---|---|
| 1.1 | 2 | 1 | 2 | 2 |
| 1.2 | 2 | 2 | 6 | 7 |
| 1.3 | 1 | 1 | 1 | 1 |
| 1.4 | 1 | 2 | 1 | 2 |
| 1.5 | 2 | 3 | 2 | 5 |
| 1.6 | 2 | 2 | 2 | 4 |
| 1.7 | 2 | 2 | 5 | 7 |
| 1.12 | 1 | 2 | 1 | 3 |
| 1.38 | 5 | 2 | 6 | 2 |
| 1.43 | 4 | 2 | 6 | 7 |
| 1.44 | 3 | 3 | 3 | 4 |
| 1.46 | 1 | 3 | 1 | 4 |
| 1.47 | 2 | 2 | 2 | 3 |

Nachweis der Selektivität bei Vorauflaufanwendung.

In der für preemrgente Test üblichen Versuchsanordnung werden eine grössere Anzahl von Pflanzensamen mit verschiedenen Aufwandmengen an Wirksubstanz der Formel I behandelt. Die Auswertung erfolgt nach dem gleichen Massstab, wie im obigen Test.

pre-emergente Wirkung:

| Wirkung | Verb. Nr. 1.1 | |
|---|---|---|
| Aufwandmenge kg AS/ha<br>Testpflanze | 0,125 | 0,06 |
| Mais | 8 | 9 |
| Weizen | 9 | 9 |
| Alopecurus myos. | 3 | 4 |
| Cyperus escul. | 3 | 4 |
| Rottboellia ex. | 5 | 5 |
| Abutilon | 2 | 2 |
| Xanthium Sp. | 2 | 2 |
| Chenopodium Sp. | 2 | 2 |
| Ipomoea | 2 | 3 |
| Sinapis | 2 | 2 |
| Galium aparine | 2 | 2 |
| Viola tricolor | 2 | 2. |

Nachweis der Herbizidwirkung nach dem Auflaufen der Pflanzen (Kontaktwirkung)
Eine Anzahl Unkräuter und Kulturpflanzen, sowohl monokotyle wie dikotyle, wurden nach dem Auflaufen im 4- bis 6-Blattstadium mit einer wässrigen Wirkstoffdispersion der Formel I in Dosierungen von 0,5 kg AS/ha gespritzt und dann bei 24° bis 26°C und 45-60% relativer Luftfeuchtigkeit gehalten. 15 Tage nach der Behandlung wird der Versuch ausgewertet und nach dem gleichen Massstab wie im pre-emergenten Versuch bewertet.

post-emergente Wirkung

Aufwandmenge: 0,5 kg Wirksubstanz/Hektar

| Verb. Nr. | Avena | Setaria | Lolium | Solanum | Sinapis | Stellaria | Phaseolus |
|---|---|---|---|---|---|---|---|
| 1.1 | 4 | 4 | 3 | 2 | 2 | 2 | 3 |

Nachweis der Selektivität bei Nachauflaufanwendung

In der gleichen Versuchsanordnung wie im vorangegangenen Beispiel werden eine grössere Anzahl von Pflanzen mit verschiedenen Aufwandmengen an Wirksubstanz behandelt. Die Auswertung erfolgte nach dem gleichen Massstab.

post-emergente Wirkung

| Wirkung | Verb. Nr. 1.1 | |
|---|---|---|
| Aufwandmange kg AS/ha<br>Testpfanze | 0,250 | 0,125 |
| Weizen | 9 | 9 |
| Mais | 7 | 9 |
| Reis trocken | 8 | 8 |
| Cyperus escul. | 3 | 4 |
| Abutilon | 3 | 4 |
| Xanthium Sp. | 3 | 4 |
| Chenopodium Sp. | 2 | 2 |
| Sinapis | 3 | 3 |
| Galium aparine | 3 | 4 |
| Viola tricolor | 2 | 3 |

Nachweis der Wuchshemmung bei tropischen Bodendeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff der Formel I als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen) ohne dass dabei die Versuchspflanzen geschädigt wurden.

Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird

das Wachstum des Getreides beurteilt. Die behandelten Pflanzen weisen im Vergleich zur unbehandelten Kontrolle eine Verringerung des Neuzuwachses (60-90% der Kontrolle), sowie teilweise eine Zunahme der Stengeldurchmesser auf.

Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerata und Cynodon dactylon im Gewächshaus angesät und nach Bedarf bewässert. Die aufgelaufenen Gräser werden wöchtentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt. Die Verbindungen der Formel I bewirken eine Reduzierung des Neuzuwachses von um 10-30% im Vergleich zur unbehandelten Kontrolle.

## Patentansprüche

**Patentansprüche für folgende Vertragsstaaten : BE, CH, DE, FR, GB, IT, LI, NL, SE**

**1.** Verbindungen der Formel IIa

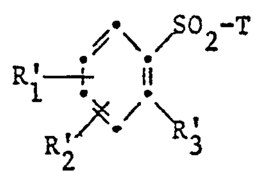

(IIa),

worin

T Hydroxyl, -OM,

$$-O\frac{M_1}{2},$$

Cl, -N=C=O oder $-NHT_4$, M ein Alkalimetallatom, $M_1$ ein Erdalkalimetallatom,

$R_1'$ Wasserstoff, Halogen, $C_1$-$C_5$-Alkyl, $C_1$-$C_4$-Halogenalkyl, $C_1$-$C_4$-Alkoxy, $-COOR_8$, $-CONR_9R_{10}$ oder $-NO_2$,

$R_2'$ Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder Alkoxyalkyl mit höchstens 4 Kohlenstoffatomen und

$R_3'$ eine Gruppe $-C(T_1)=C(T_2)(T_3)$ bedeuten,

$T_1$ Wasserstoff,

$T_2$ ein- oder mehrfach durch Fluor substituiertes $C_1$-$C_8$-Alkyl,

$T_3$ Wasserstoff oder gegebenenfalls durch Fluoratome substituiertes $C_1$-$C_5$-Alkyl,

$T_4$ Wasserstoff, -CO-NH-$C_1$-$C_4$-Alkyl oder -COO-$C_1$-$C_4$-Alkyl oder -COO-Phenyl,

$R_8$ $C_1$-$C_4$-Alkyl, $C_1$-$C_4$-Halogenalkyl oder $C_2$-$C_6$-Alkoxyalkyl, und

$R_9$ und $R_{10}$ unabhängig voneinander Wasserstoff oder $C_1$-$C_3$-Alkyl bedeuten, wobei durch $T_2$ und $T_3$ dargestellte Alkylgruppen zusammen höchstens 8 Kohlenstoffatome aufweisen.

**2.** Verbindungen der Formel IIa gemäss Anspruch 1, dadurch gekennzeichnet, dass T eine Aminogruppe darstellt.

**3.** 2-(3,3,3-Trifluor-1-propen-1-yl)-phenylsulfonamid nach Anspruch 1.

**4.** 2-(3,3-Difluor-1-buten-1-yl)-phenylsulfonamid nach Anspruch 1.

**5.** Verfahren zur Herstellung der Verbindungen der Formel IIa gemäss Anspruch 1, dadurch gekennzeichnet, dass man ein Amin der Formel VIII

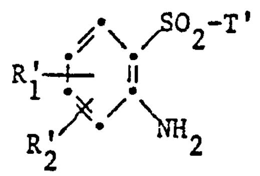

(VIII)

zu einem Diazoniumsalz der Formel IX

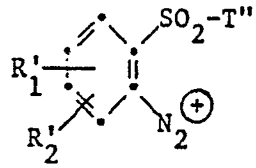

(IX)

diazotiert, dieses in Gegenwart von Palladium-Katalysatoren, die unter den Reaktionsbedingungen Pd-(O)-Verbindungen bilden, und gegebenenfalls in Gegenwart einer Base mit einer Verbindung der Formel X

$H - R_3'$ (X)

zu einer Verbindung der Formel XI

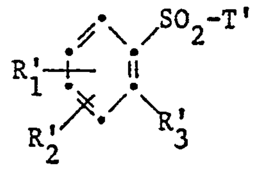

(XI)

umsetzt, die Verbindung der Formel XI in an sich bekannter Weise durch Behandlung mit einem Chlorierungsmittel,
in das entsprechende Sulfonylchlorid überführt, dieses mit Ammoniak behandelt und so erhaltene Verbindungen der Formel IIa, worin T für $-NH_2$ steht, gegebenenfalls in Verbindungen der Formel IIa, worin $T_4$ nicht Wasserstoff bedeutet, überführt, indem man die erhaltenen Sulfonamide mit den Acylierungsmitteln $O=C=N-C_1-C_4$-Alkyl, $ClCONH-C_1-C_4$-Alkyl, $-ClCOO-C_1-C_4$-Alkyl, ClCOO-Phenyl oder mit $C_1-C_4$-Alkylisocyanaten, wobei T' OH, OM oder

$$O\frac{M_1}{2}$$

und T" OM oder

$$O\frac{M_1}{2}$$

bedeuten und M, $M_1$, $R_1'$, $R_2$, $R_3'$ die unter Formel IIa angegebene Bedeutung haben, umsetzt.

**6.** Verfahren zur Herstellung der Verbindungen der Formel IIa gemäss Anspruch 1, worin $R_1'$ und $R_2'$ nicht Brom oder Jod bedeuten, dadurch gekennzeichnet, dass man eine Verbindung der Formel XII

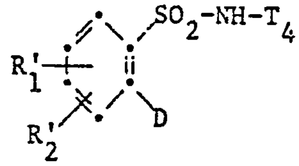

(XII)

worin $R_1'$ , $R_2'$ und $T_4$ die unter Formel IIa angegebene Bedeutung haben und D Brom oder Jod bedeutet, in Gegenwart einer gegebenenfalls arsen- oder phosphorhaltigen Palladium-Verbindung als Katalysator und einer Base mit einem Olefin der Formel X

$H\text{-}R_3'$ (X)

worin $R_3'$ die im Anspruch 1 angegeben Bedeutung hat, umsetzt.

**Patentansprüche für folgenden Vertragsstaat : AT**

**1.** Verfahren zur Herstellung der Verbindungen der Formel IIa

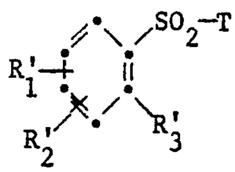

(IIa),

worin

| | |
|---|---|
| T | Hydroxyl, -OM, $-O\frac{M_1}{2}$, Cl, -N=C=O oder $-NHT_4$, M ein Alkalimetallatom, $M_1$ ein Erdalkalimetallatom, |
| $R_1'$ | Wasserstoff, Halogen, $C_1$-$C_5$-Alkyl, $C_1$-$C_4$-Halogenalkyl, $C_1$-$C_4$-Alkoxy, $-COOR_8$, $-CONR_9R_{10}$ oder $-NO_2$, |
| $R_2'$ | Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy oder Alkoxyalkyl mit höchstens 4 Kohlenstoffatomen und |
| $R_3'$ | eine Gruppe $-C(T_1)=C(T_2)(T_3)$ bedeuten, |
| $T_1$ | Wasserstoff, |
| $T_2$ | ein- oder mehrfach durch Fluor substituiertes $C_1$-$C_8$-Alkyl, |
| $T_3$ | Wasserstoff oder gegebenenfalls durch Fluoratome substituiertes $C_1$-$C_5$-Alkyl, |
| $T_4$ | Wasserstoff, -CO-NH-$C_1$-$C_4$-Alkyl oder -COO-$C_1$-$C_4$-Alkyl oder -COO-Phenyl, |
| $R_8$ | $C_1$-$C_4$-Alkyl, $C_1$-$C_4$-Halogenalkyl oder $C_2$-$C_6$-Alkoxyalkyl, und |
| $R_9$ und $R_{10}$ | unabhängig voneinander Wasserstoff oder $C_1$-$C_3$-Alkyl bedeuten, wobei durch $T_2$ und $T_3$ dargestellte Alkylgruppen zusammen höchstens 8 Kohlenstoffatome aufweisen, dadurch gekennzeichnet, dass man ein Amin der Formel VIII |

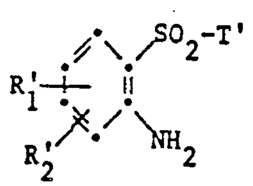

(VIII)

zu einem Diazoniumsalz der Formel IX

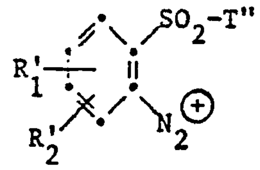

(IX)

diazotiert, dieses in Gegenwart von Palladium-Katalysatoren, die unter den Reaktionsbedingungen Pd-(O)-Verbindungen bilden, und gegebenenfalls in Gegenwart einer Base mit einer Verbindung der Formel X

$H - R_3'$ (X)

zu einer Verbindung der Formel XI

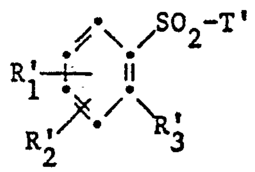

(XI)

umsetzt, die Verbindung der Formel XI in an sich bekannter Weise durch Behandlung mit einem Chlorierungsmittel,
in das entsprechende Sulfonylchlorid überführt, dieses mit Ammoniak behandelt und so erhaltene Verbindungen der Formel IIa, worin T für $-NH_2$ steht, gegebenenfalls in Verbindungen der Formel IIa, worin $T_4$ nicht Wasserstoff bedeutet, überführt, indem man die erhaltenen Sulfonamide mit dem Acylierungsmitteln $O=C=N-C_1-C_4$-Alkyl, $ClCONH-C_1-C_4$-Alkyl, $-ClCOO-C_1-C_4$-Alkyl, ClCOO-Phenyl oder mit $C_1-C_4$-Alkylisocyanaten, wobei T′ OH, OM oder

$$O\frac{M_1}{2}$$

und T″ OM oder

$$O\frac{M_1}{2}$$

bedeuten und M, $M_1$, $R_1'$, $R_2$, $R_3'$ die unter Formel IIa angegebene Bedeutung haben, umsetzt.

**2.** Verfahren gemäss Anspruch 1, dadurch gekennzeichnet, dass T eine Aminogruppe darstellt.

**3.** Verfahren gemäss Anspruch 1 zur Herstellung von 2-(3,3,3-Trifluor-1-propen-1-yl)-phenylsulfonamid.

**4.** Verfahren gemäss Anspruch 1 zur Herstellung von 2-(3,3-Difluor-1-buten-1-yl)-phenylsulfonamid.

**5.** Verfahren zur Herstellung der Verbindungen der Formel IIa gemäss Anspruch 1, worin $R_1'$ und $R_2'$ nicht Brom oder Jod bedeuten, dadurch gekennzeichnet, dass man eine Verbindung der Formel XII

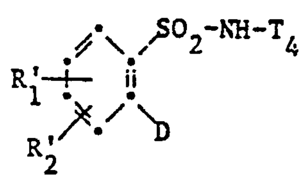

(XII)

worin $R_1'$, $R_2'$ und $T_4$ die unter Formel IIa angegebene Bedeutung haben und D Brom oder Jod

bedeutet, in Gegenwart einer gegebenenfalls arsen- oder phosphorhaltigen Palladium-Verbindung als Katalysator und einer Base mit einem Olefin der Formel X

$H\text{-}R'_3$ (X)

worin $R'_3$ die im Anspruch 1 angegebene Bedeutung hat, umsetzt.

**Claims**

**Claims for the following Contracting States : BE, CH, DE, FR, GB, IT, LI, NL, SE**

1. A compound of the formula IIa

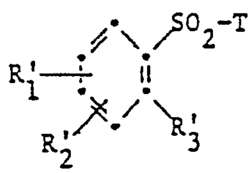

(IIa)

in which
T is hydroxyl, -OM,

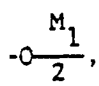

Cl, -N=C=O or $-NHT_4$, M is an alkali metal atom and $M_1$ is an alkaline earth metal atom,
$R'_1$ is hydrogen, halogen, $C_1$-$C_5$alkyl, $C_1$-$C_4$haloalkyl, $C_1$-$C_4$alkoxy, $-COOR_8$, $-CONR_9R_{10}$ or $-NO_2$,
$R'_2$ is hydrogen, halogen, $C_1$-$C_4$alkyl, $C_1$-$C_4$haloalkyl, $C_1$-$C_4$alkoxy, $C_1$-$C_4$haloalkoxy or alkoxyalkyl having not more than 4 carbon atoms,
$R'_3$ is a $-C(T_1)=C(T_2)(T_3)$ group,
$T_1$ is hydrogen,
$T_2$ is $C_1$-$C_8$alkyl which is substituted by one or more fluorine atoms,
$T_3$ is hydrogen or $C_1$-$C_5$alkyl which is unsubstituted or substituted by fluorine atoms,
$T_4$ is hydrogen, -CO-NH-$C_1$-$C_4$alkyl, -COO-$C_1$-$C_4$alkyl or -COO-phenyl,
$R_8$ is $C_1$-$C_4$alkyl, $C_1$-$C_4$haloalkyl or $C_2$-$C_6$alkoxyalkyl, and
$R_9$ and $R_{10}$, each independently of the other, are hydrogen or $C_1$-$C_3$alkyl, the alkyl groups $T_2$ and $T_3$ together containing not more than 8 carbon atoms.

2. A compound of the formula IIa according to claim 1, wherein T is an amino group.

3. 2-(3,3,3-Trifluoro-1-propen-1-yl)phenylsulfonamide according to claim 1.

4. 2-(3,3-Difluoro-1-buten-1-yl)phenylsulfonamide according to claim 1.

5. A process for the preparation of a compound of the formula IIa according to claim 1, which comprises diazotising an amine of the formula VIII

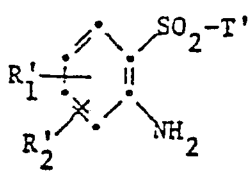

(VIII)

to a diazonium salt of the formula IX

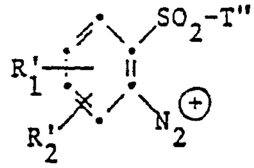 (IX),

reacting said diazonium salt with a compound of the formula X

H - $R_3'$  (X)

in the presence of a palladium catalyst which forms a Pd(O) compound under the reaction conditions, and if desired in the presence of a base, to a compound of the formula XI

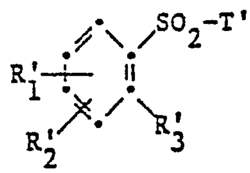 (XI),

converting the compound of the formula XI, in a manner known per se, into the corresponding sulfonyl chloride by treatment with a chlorinating agent, treating the sulfonyl chloride with ammonia and, if desired, converting the compound of formula IIa thus obtained, in which T is $-NH_2$, into a compound of the formula IIa, in which $T_4$ is not hydrogen, by reacting the sulfonamides obtained with an acylating agent $O=C=N-C_1-C_4$alkyl, $ClCONH-C_1-C_4$alkyl, $ClCOO-C_1-C_4$alkyl, ClCOO-phenyl, or with $C_1-C_4$-alkylisocyanate, and T' is OH, OM or

$$O\frac{M_1}{2},$$

T" is OM or

$$O\frac{M_1}{2},$$

and M, $M_1$, $R_1'$ , $R_2$ and $R_3'$ are as defined for formula IIa.

**6.** A process for the preparation of a compound of the formula IIa according to claim 1, in which $R_1'$ and $R_2'$ are not bromine or iodine, which comprises reacting a compound of the formula XII

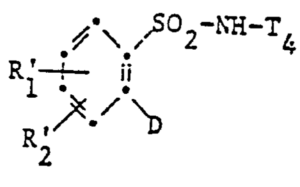 (XII)

in which $R_1'$ , $R_2'$ and $T_4$ are as defined for formula IIa and D is bromine or iodine, with an olefin of the formula X

H - $R_3'$  (X)

in which $R_3'$ is as defined in claim 1, in the presence of a palladium catalyst which may contain arsenic or phosphorus and of a base.

**Claims for the following Contracting State : AT**

**1.** A process for the preparation of a compound of the formula IIa

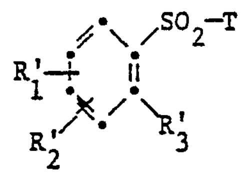

(IIa)

in which
T is hydroxyl, -OM,

$$-O\frac{M_1}{2},$$

Cl, -N=C=O or $-NHT_4$, M is an alkali metal atom and $M_1$ is an alkaline earth metal atom,
$R_1'$ is hydrogen, halogen, $C_1$-$C_5$alkyl, $C_1$-$C_4$haloalkyl, $C_1$-$C_4$alkoxy, $-COOR_8$, $-CONR_9R_{10}$ or $-NO_2$,
$R_2'$ is hydrogen, halogen, $C_1$-$C_4$alkyl, $C_1$-$C_4$haloalkyl, $C_1$-$C_4$alkoxy, $C_1$-$C_4$haloalkoxy or alkoxyalkyl having not more than 4 carbon atoms,
$R_3'$ is a $-C(T_1)=C(T_2)(T_3)$ group,
$T_1$ is hydrogen,
$T_2$ is $C_1$-$C_8$alkyl which is substituted by one or more fluorine atoms,
$T_3$ is hydrogen or $C_1$-$C_5$alkyl which is unsubstituted or substituted by fluorine atoms,
$T_4$ is hydrogen, -CO-NH-$C_1$-$C_4$alkyl, -COO-$C_1$-$C_4$alkyl or -COO-phenyl,
$R_8$ is $C_1$-$C_4$alkyl, $C_1$-$C_4$haloalkyl or $C_2$-$C_6$alkoxyalkyl, and
$R_9$ and $R_{10}$, each independently of the other, are hydrogen or $C_1$-$C_3$alkyl,
the alkyl groups $T_2$ and $T_3$ together containing not more than 8 carbon atoms, which comprises diazotising an amine of the formula VIII

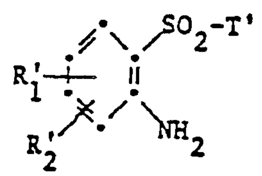

(VIII)

to a diazonium salt of the formula IX

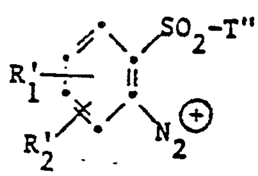

(IX),

reacting said diazonium salt with a compound of the formula X

$H - R_3'$ (X)

in the presence of a palladium catalyst which forms a Pd(O) compound under the reaction conditions, and if desired in the presence of a base, to a compound of the formula XI

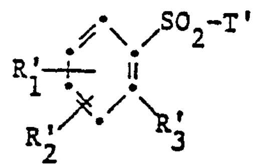

(XI),

converting the compound of the formula XI, in a manner known per se, into the corresponding sulfonyl chloride by treatment with a chlorinating agent, treating the sulfonyl chloride with ammonia and, if desired, converting the compound of formula IIa thus obtained, in which T is $-NH_2$, into a compound of the formula IIa, in which $T_4$ is not hydrogen, by reacting the sulfonamides obtained with an acylating agent $O=C=N-C_1-C_4$alkyl, $ClCONH-C_1-C_4$alkyl, $ClCOO-C_1-C_4$alkyl, ClCOO-phenyl, or with $C_1$-$C_4$-alkylisocyanate, and T' is OH, OM or

$$O-\frac{M_1}{2},$$

T" is OM or

$$O-\frac{M_1}{2},$$

and M, $M_1$, $R_1'$, $R_2$ and $R_3'$ are as defined for formula IIa.

2. A process according to claim 1, wherein T is an amino group.

3. A process according to claim 1 for the preparation of 2-(3,3,3-trifluoro-1-propen-1-yl)phenylsulfonamide.

4. A process according to claim 1 for the preparation of 2-(3,3-difluoro-1-buten-1-yl)phenylsulfonamide.

5. A process for the preparation of a compound of the formula IIa according to claim 1, in which $R_1'$ and $R_2'$ are not bromine or iodine, which comprises reacting a compound of the formula XII

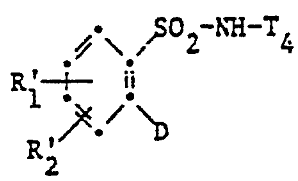

(XII)

in which $R_1'$, $R_2'$ and $T_4$ are as defined for formula IIa and D is bromine or iodine, with an olefin of the formula X

$H-R_3'$ (X)

in which $R_3'$ is as defined in claim 1, in the presence of a palladium catalyst which may contain arsenic or phosphorus and of a base.

## Revendications

**Revendication pour les Etats contractants suivants : BE, CH, DE, FR, GB, IT, LI, NL, SE**

1. Composés de formule IIa

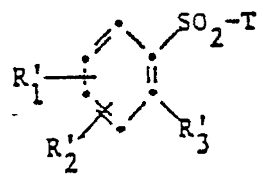

(IIa),

dans laquelle

T représente un groupe hydroxy, -OM,

$$-O\frac{M_1}{2},$$

CL, -N=C=O, ou $-NHT_4$, M représente un atome de métal alcalin, $M_1$ un atome de métal alcalino-terreux,

$R'_1$ représente l'hydrogène, un halogène, un groupe alkyle en C1-C5, halogénoalkyle en C1-C4, alcoxy en C1-C4, $-COOR_8$, $-CONR_9R_{10}$ ou $-NO_2$,

$R'_2$ représente l'hydrogène, un halogène, un groupe alkyle en C1-C4, halogénoalkyle en C1-C4, alcoxy en C1-C4, halogénoalcoxy en C1-C4 ou alcoxyalkyle contenant au maximum 4 atomes de carbone et

$R'_3$ représente un groupe $-C(T_1)=C(T_2)(T_3)$,

$T_1$ représente l'hydrogène,

$T_2$ représente un groupe alkyle en C1-C8 mono- ou polysubstitué par le fluor,

$T_3$ représente l'hydrogène ou un groupe alkyle en C1-C5 éventuellement substitué par des atomes de fluor,

$T_4$ représente l'hydrogène, un groupe -CO-NH-alkyle en C1-C4 ou -COO-alkyle en C1-C4 ou -COO-phényle,

$R_8$ représente un groupe alkyle en C1-C4, halogénoalkyle en C1-C4 ou alcoxyalkyle en C2-C6 et

$R_9$ et $R_{10}$ représentent chacun, indépendamment l'un de l'autre, l'hydrogène ou un groupe alkyle en C1-C3,

les groupes alkyle représentés par $T_2$ et $T_3$ contenant ensemble, au maximum, 8 atomes de carbone.

**2.** Composés de formule IIa selon revendication 1, caractérisés en ce que T représente un groupe amino.

**3.** Le 2-(3,3,3-trifluoro-1-propène-1-yl)-phénylsulfonamide selon revendication 1.

**4.** Le 2-(3,3-difluoro-1-butène-1-yl)-phénylsulfonamide selon revendication 1.

**5.** Procédé de préparation des composés de formule IIa de la revendication 1, caractérisé en ce que l'on diazote une amine de formule VIII

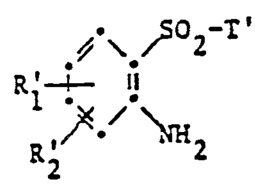

(VIII)

ce qui donne un sel de diazonium de formule IX

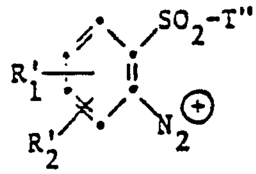

(IX)

qu'on convertit en présence de catalyseurs au palladium formant, dans les conditions de la réaction, des dérivés de Pd (O), et éventuellement en présence d'une base, par réaction avec un composé de formule X

H -R'$_3$ (X)

en un composé de formule XI

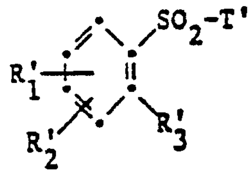

(XI)

qu'on convertit de manière connue en soi, par traitement à l'aide d'un agent chlorant, en le chlorure de sulfonyle correspondant, qu'on traite par l'ammoniac, ce qui donne les composés de formule IIa dans laquelle T représente -NH$_2$ qu'on convertit, le cas échéant, en composés de formule IIa dans laquelle T$_4$ a une signification autre que l'hydrogène, en faisant réagir les sulfonamides ainsi obtenus avec les agents acylants O = C = N-alkyle en C1-C4, ClCONH-alkyle en C1-C4, -ClCOO-alkyle en C1-C4, ClCOO-phényle ou avec des isocyanates d'alkyle en C1-C4, T' représentant OH, OM ou

$$O\frac{M_1}{2}$$

et T" représentant OM ou

$$O\frac{M_1}{2} ,$$

et M, M$_1$, R'$_1$, R$_2$, R'$_3$ ayant les significations indiquées en référence à la formule IIa.

**6.** Procédé de préparation des composés de formule IIa de la revendication 1, dans laquelle R'$_1$ et R'$_2$ ne représentent pas le brome ou l'iode, caractérisé en ce que l'on fait réagir un composé de formule XII

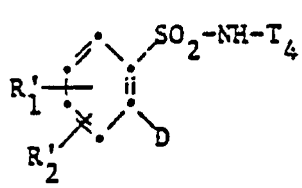

(XII)

dans laquelle R'$_1$ R'$_2$ et T$_4$ ont les significations indiquées en référence à la formule IIa et D représente le brome ou l'iode, en présence d'un composé du palladium contenant éventuellement de l'arsenic ou du phosphore et servant de catalyseur, et d'une base, avec une oléfine de formule X

H - R'₃ (X)

dans laquelle $R'_3$ a les significations indiquées dans la revendication 1.

**Revendications pour l'Etat contractant suivant : AT**

**1.** Procédé de préparation des composés de formule IIa

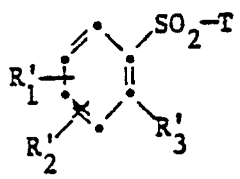

(IIa),

dans laquelle

T représente un groupe hydroxy, -OM,

$$-O\frac{M_1}{2} ,$$

Cl, -N=C=O, ou $-NHT_4$, M représente un atome de métal alcalin, $M_1$ un atome de métal alcalino-terreux,

$R'_1$ représente l'hydrogène, un halogène, un groupe alkyle en C1-C5, halogénoalkyle en C1-C4, alcoxy en C1-C4, $-COOR_8$, $-CONR_9R_{10}$ ou $-NO_2$,

$R'_2$ représente l'hydrogène, un halogène, un groupe alkyle en C1-C4, halogénoalkyle en C1-C4, alcoxy en C1-C4, halogénoalcoxy en C1-C4 ou alcoxyalkyle contenant au maximum 4 atomes de carbone et

$P'_3$ représente un groupe $-C(T_1)=C(T_2)(T_3)$,

$T_1$ représente l'hydrogène,

$T_2$ représente un groupe alkyle en C1-C8 mono- ou polysubstitué par le fluor,

$T_3$ représente l'hydrogène ou un groupe alkyle en C1-C5 éventuellement substitué par des atomes de fluor,

$T_4$ représente l'hydrogène, un groupe -CO-NH-alkyle en C1-C4 ou -COO-alkyle en C1-C4 ou -COO-phényle,

$R_8$ représente un groupe alkyle en C1-C4, halogénoalkyle en C1-C4 ou alcoxyalkyle en C2-C6 et

$R_9$ et $R_{10}$ représentent chacun, indépendamment l'un de l'autre, l'hydrogène ou un groupe alkyle en C1-C3,

les groupes alkyle représentés par $T_2$ et $T_3$ contenant ensemble, au maximum, 8 atomes de carbone, caractérisé en ce que l'on diazote une amine de formule VIII

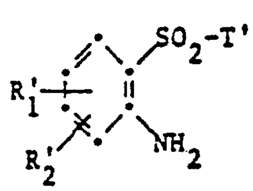

(VIII)

ce qui donne un sel de diazonium de formule IX

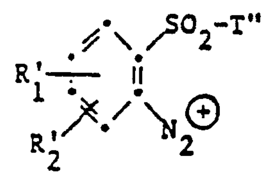

(IX)

qu'on convertit en présence de catalyseurs au palladium formant, dans les conditions de la réaction, des dérivés de Pd (O), et éventuellement en présence d'une base, par réaction avec un composé de formule X

H - $R'_3$ (X)

en un composé de formule XI

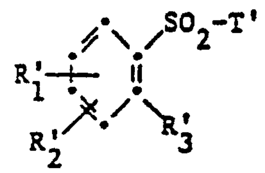

(XI)

qu'on convertit de manière connue en soi, par traitement à l'aide d'un agent chlorant, en le chlorure de sulfonyle correspondant, qu'on traite par l'ammoniac, ce qui donne les composés de formule IIa dans laquelle T représente $-NH_2$ qu'on convertit, le cas échéant, en composés de formule IIa dans laquelle $T_4$ a une signification autre que l'hydrogène, en faisant réagir les sulfonamides ainsi obtenus avec les agents acylants O=C=N-alkyle en C1-C4, ClCONH-alkyle en C1-C4, -ClOOO-alkyle en C1-C4, ClCOO-phényle ou avec des isocyanates d'alkyle en C1-C4, T' représentant OH, OM ou

$$O\frac{M_1^-}{2}$$

et T" représentant OM ou

$$O\frac{M_1}{2},$$

, et M, $M_1$, $R'_1$, $R_2$, $R'_3$ ayant les significations indiquées en référence à la formule IIa.

2. Procédé selon la revendication 1, caractérisé en ce que T représente un groupe amino.

3. Procédé selon la revendication 1, pour la préparation du 2-(3,3,3-trifluoro-1-propène-1-yl)-phénylsulfonamide.

4. Procédé selon la revendication 1, pour la préparation du 2-(3,3-difluoro-1-butène-1-yl)-phénylsulfonamide.

5. Procédé de préparation des composés de formule IIa de la revendication 1, dans laquelle $R'_1$ et $R'_2$ ne représentent pas le brome ou l'iode, caractérisé en ce que l'on fait réagir un composé de formule XII

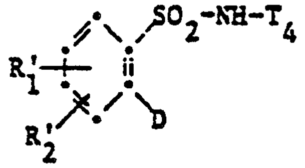

(XII)

dans laquelle R'$_1$, R'$_2$ et $T_4$ ont les significations indiquées en référence à la formule IIa et D représente le brome ou l'iode, en présence d'un composé du palladium contenant éventuellement de l'arsenic ou du phosphore et servant de catalyseur, et d'une base, avec une oléfine de formule X

H - R'$_3$ (X)

dans laquelle R'$_3$ a les significations indiquées dans la revendication 1.